# EUROPEAN PATENT APPLICATION

(11) **EP 4 582 075 A1**
(43) Date of publication of application: **09.07.2025**
(21) Application number: 23860791.5
(22) Date of filing: 24.08.2023
(51) Int. Cl.: A61K 8/34, A61K 8/73, A61K 8/9728, A61Q 19/00, A61K 31/047, A61K 31/733, A61K 36/06, A61P 17/00

(54) **TOPICAL SKIN COMPOSITION CONTAINING 1,3-PROPANEDIOL AS ACTIVE INGREDIENT FOR MAINTAINING MICROBIOME BALANCE IN SKIN**

(30) Priority: 01.09.2022 KR 20220110525
(71) Applicant: Activon Co., Ltd., Cheongju-si, Chungcheongbuk-do 28104 (KR)
(72) Inventor: KIM, Myo Deok, Pyeongtaek-si Gyeonggi-do 18019 (KR); KIM, Dae Gun, Hanam-si Gyeonggi-do 12942 (KR); CHO, Myung Chan, Seoul 06310 (KR); PARK, Byung Gyu, Suwon-si Gyeonggi-do 16668 (KR); JUNG, Sung Won, Yongin-si Gyeonggi-do 16909 (KR); CHO, Youn Ki, Yongin-si Gyeonggi-do 17005 (KR)
(74) Representative: Vossius & Partner Patentanwälte Rechtsanwälte mbB
(86) International application number: PCT/KR2023/012605
(87) International publication number: WO 2024/049114

(57) **Abstract**

The present disclosure relates to a composition for skin external use for maintaining the balance of the skin microbiome, containing 1,3-propanediol as an active ingredient and optionally a boosting ingredient. The composition can be formulated into a cosmetic composition or a pharmaceutical composition for application to the outside of the skin. The composition for skin external use according to the present disclosure can promote the growth of beneficial skin bacteria, suppress the growth of harmful skin bacteria, maintain the balance of the skin microbiome, thereby improve and strengthen the barrier function against external irritants of the skin. It can also help to make skin tissue dense, improve skin texture, and maintain overall skin health.

## Description

### TECHNICAL FIELD

### CROSS-REFERENCES TO RELATED APPLICATIONS

This application claims the benefit of priority based on Korean Patent Application No. 10-2022-0110525 filed on September 1, 2022, and Korean Patent Application No. 10-2023-0111037 filed on August 24, 2023, and the entire contents of the Korean patent application are incorporated herein by reference.

### TECHNICAL FIELD

The present disclosure relates to a composition for skin external use for maintaining the balance of the skin microbiome, preferably a cosmetic composition, containing 1,3-propanediol as an active ingredient.

### BACKGROUND ART

The term "microbiome" is a compound word of "microbiota" and "genome," and can be defined as a microbial community including microorganisms and their entire genetic information that inhabit or coexist in any environments such as humans, animals, plants, soil, sea, lakes, cliffs, and atmosphere. Recently, it also refers to microbial communities in various environments. In addition, studies on various microbiomes such as plant microbiome, animal microbiome, environmental microbiome, human microbiome, intestinal microbiome, and skin microbiome are being conducted, and among them, studies on the human microbiome are being conducted the most.

The human body is a complex and highly active biological ecosystem, with diverse microorganisms present in areas such as the human stomach and skin. More than 500 to 1,000 different species of microorganisms coexist in the human stomach and skin, forming microbial flora.

Microorganisms present on the skin surface affect physiological functions such as human growth, nutrition, immunity, and resistance to pathogenicity, and play a role in blocking the invasion of external microorganisms, particularly pathogenic microorganisms.

However, since microorganisms present on the skin include not only beneficial bacteria but also harmful bacteria, it is necessary to maintain the balance of the skin microbiome by promoting the growth of beneficial bacteria while suppressing the growth of harmful bacteria for skin health.

### DISCLOSURE OF THE INVENTION

### TECHNICAL PROBLEM

The present disclosure is to provide a cosmetic composition capable of maintaining the balance of the skin microbiome by increasing beneficial bacteria and decreasing harmful bacteria in the skin.

### TECHNICAL SOLUTION

In one embodiment, the present disclosure provides a composition for skin external use for maintaining the balance of the skin microbiome, a cosmetic composition, containing 1,3-propanediol as an active ingredient.

In the present disclosure, maintaining the balance of the microbiome may refer to increasing beneficial bacteria while decreasing harmful bacteria in the skin.

In the present disclosure, the beneficial bacteria may be one or more selected from the group consisting of Staphylococcus epidermidis, streptococcus pneumoniae, lactobacillus acidophilus, lactobacillus rhamnosus, and bifidobacterium bifidum, and the harmful bacteria may be one or more selected from the group consisting of Staphylococcus aureus and cutibacterium acnes.

In the cosmetic composition according to the present disclosure, the effective concentration of 1,3-propanediol may range from 0.1 to 20 wt% based on the total weight of the composition, preferably from 0.2 to 10 wt%.

In another embodiment, the present disclosure provides a cosmetic composition for maintaining the balance of the skin microbiome, containing 1,3-propanediol and optionally a boosting ingredient.

In the present disclosure, the boosting ingredient may be at least one selected from the group consisting of fructan and yeast extract.

In the present disclosure, the effective concentration of the boosting ingredient may range from 0.06 to 0.25 wt% based on the total weight of the composition, preferably from 0.09 to 0.12 wt%.

In the present disclosure, the combination ratio of 1,3-propanediol and the boosting ingredient may range from 6 to 25:1 by weight, preferably from 12.5 to 16.7:1 by weight.

In the present disclosure, the cosmetic composition may be formulated into a form selected from the group consisting of solution, suspension, emulsion, paste, gel, cream, lotion, powder, soap, surfactant-containing cleanser, oil, microcapsule, microgranule, ionic vesicle dispersion, non-ionic vesicle dispersion, powder foundation, emulsion foundation, wax foundation, stick, mask pack, and spray.

In another embodiment, the present disclosure provides a method for maintaining or restoring the balance of the skin microbiome by topically applying to the skin a skin external composition, particularly a cosmetic composition, containing as an active ingredient 1,3-propanediol or a combination of 1,3-propanediol and a boosting ingredient.

In another embodiment, the present disclosure provides the use of 1,3-propanediol or a combination of 1,3-propanediol and a boosting ingredient as an active ingredient for maintaining the balance of the skin microbiome in a skin external composition, particularly a cosmetic composition.

### ADVANTAGEOUS EFFECTS

The present disclosure can provide a skin external composition that is beneficial for skin health by maintaining and/or restoring the balance of the skin microbiome by increasing beneficial bacteria while suppressing the growth and/or activity of harmful bacteria in the skin.

In addition, the skin external composition according to the present disclosure, by including 1,3-propanediol as an active ingredient, promotes the growth of beneficial skin bacteria and suppresses the growth of harmful skin bacteria to maintain the balance of the skin microbiome, thereby improving and strengthening the barrier function against external irritants of the skin, and making it highly valuable for practical use. In addition, it may help to make the skin tissue firm, improve skin texture, and maintain overall skin health, and has the advantage of contributing to improving the quality of life by boosting the user's confidence in their skin.

### BRIEF DESCRIPTION OF THE DRAWINGS

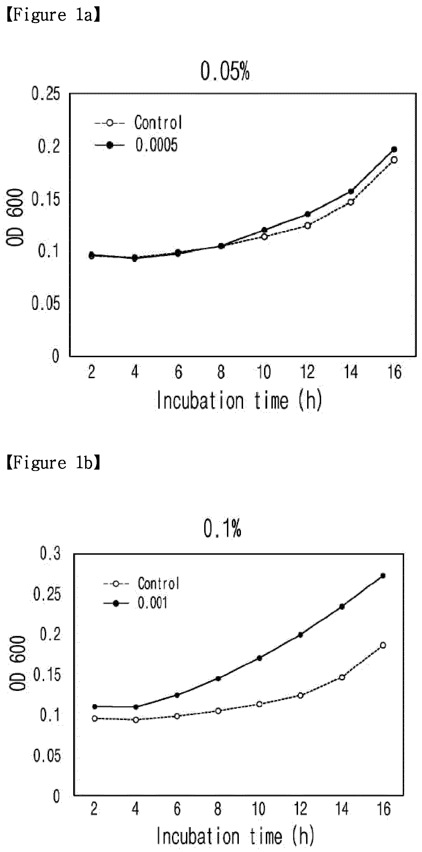

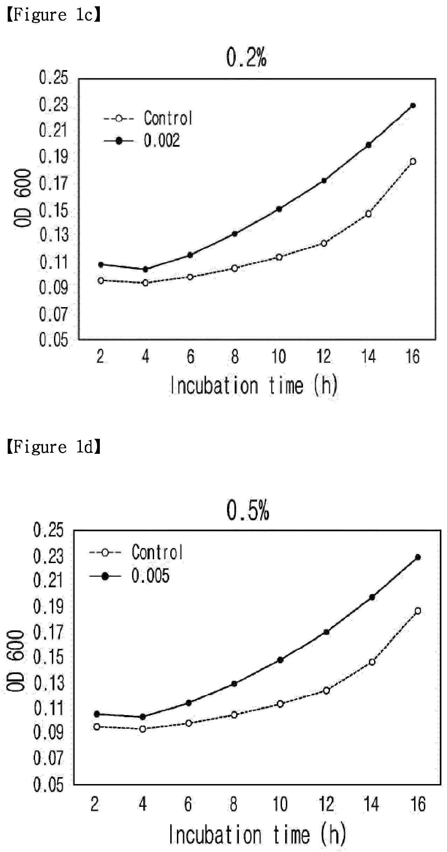

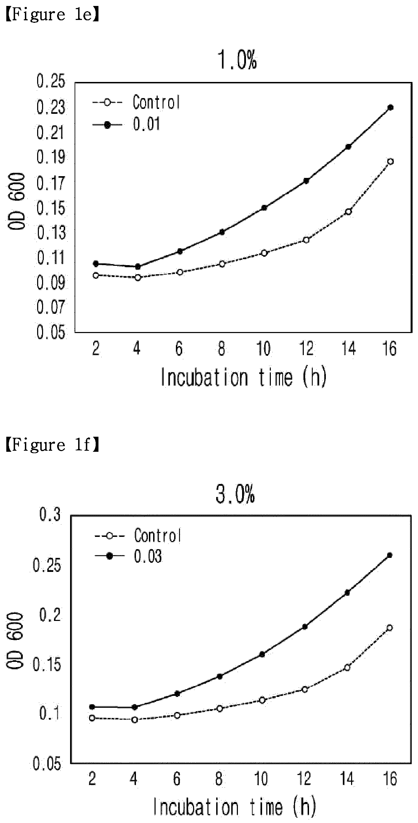

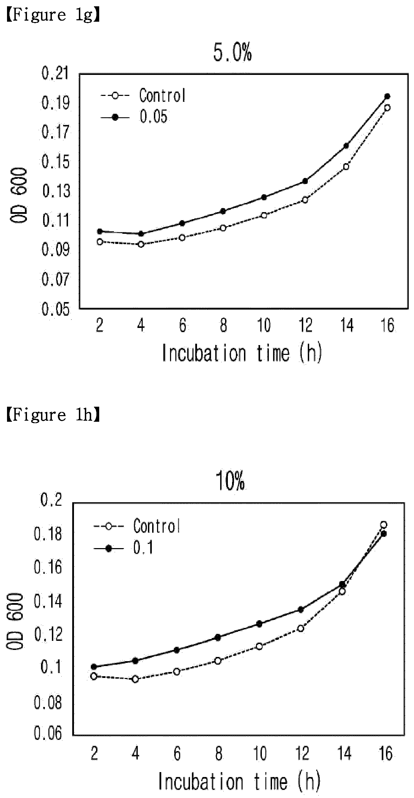

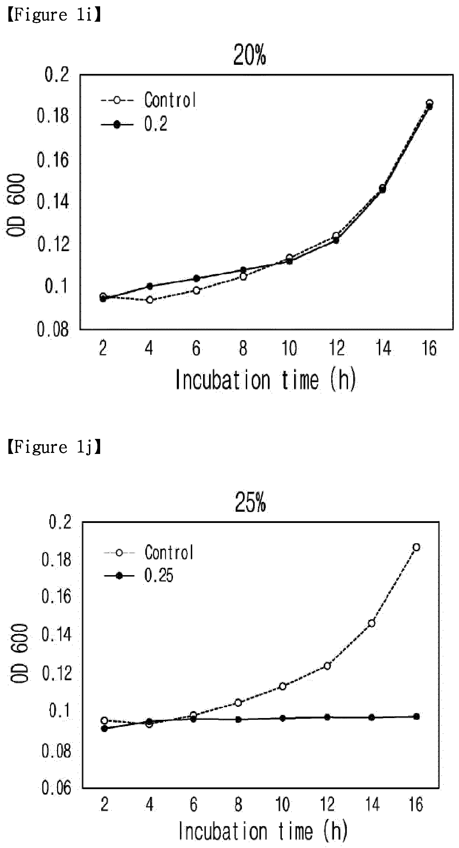

FIGS. 1A to 1J illustrate the results of analyzing the effect of 1,3-propanediol on the growth of the beneficial skin bacteria StaphylococcusStaphylococcus epidermidis ATCC 12228. FIGS. 1A to 1J illustrate growth trends through changes in absorbance (OD) values over incubation time of beneficial skin bacteria compared to the control group for each treatment concentration of 1,3-propanediol: FIG. 1A: 1,3-propanediol treatment concentration 0.05%; FIG. 1B: 1,3-propanediol treatment concentration 0.1%; FIG. 1C: 1,3-propanediol treatment concentration 0.2%; FIG. 1D: 1,3-propanediol treatment concentration 0.5%; FIG. 1E: 1,3-propanediol treatment concentration 1.0%; FIG. 1F: 1,3-propanediol treatment concentration 3.0%; FIG. 1G: 1,3-propanediol treatment concentration 5.0%; FIG. 1H: 1,3-propanediol treatment concentration 10.0%; FIG. 1I: 1,3-propanediol treatment concentration 20.0%; FIG. 1J: 1,3-propanediol treatment concentration 25.0%.

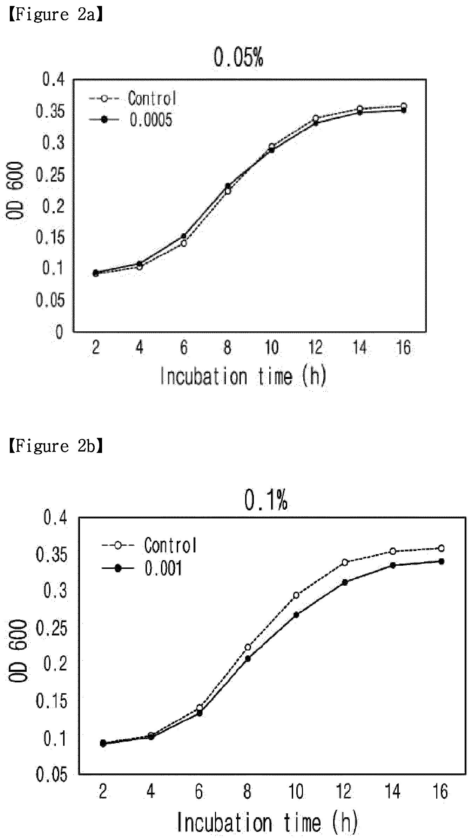

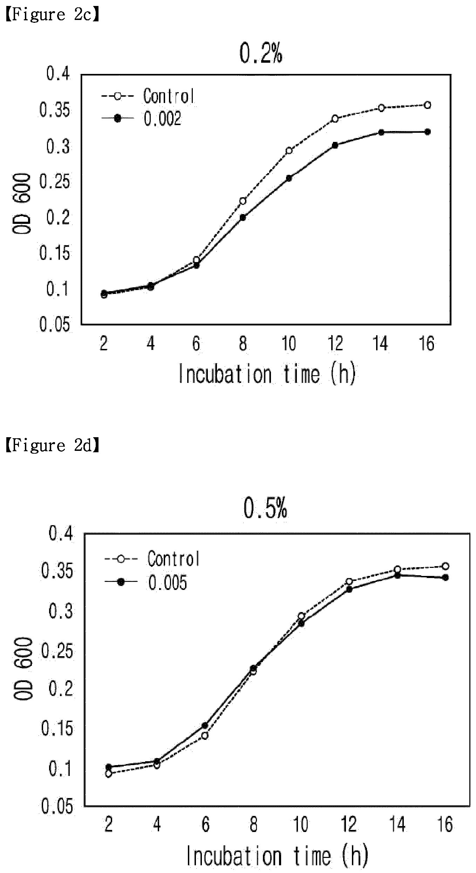

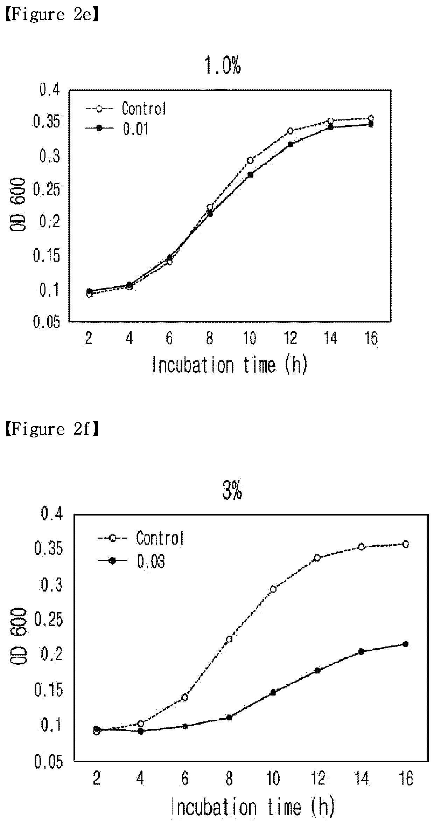

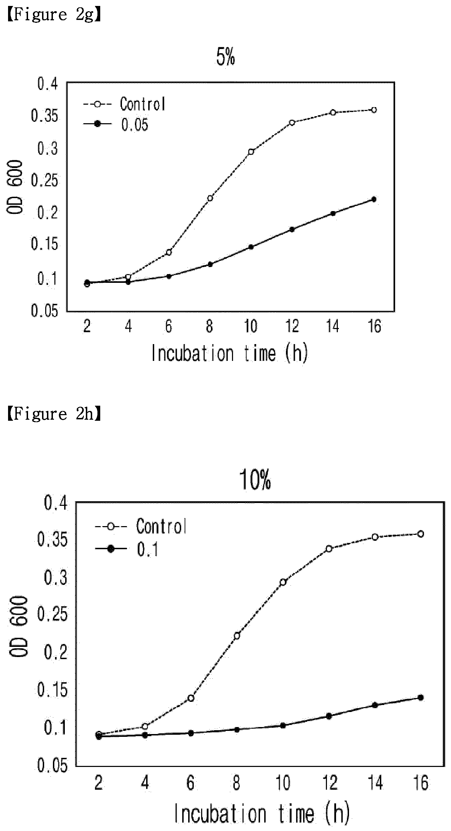

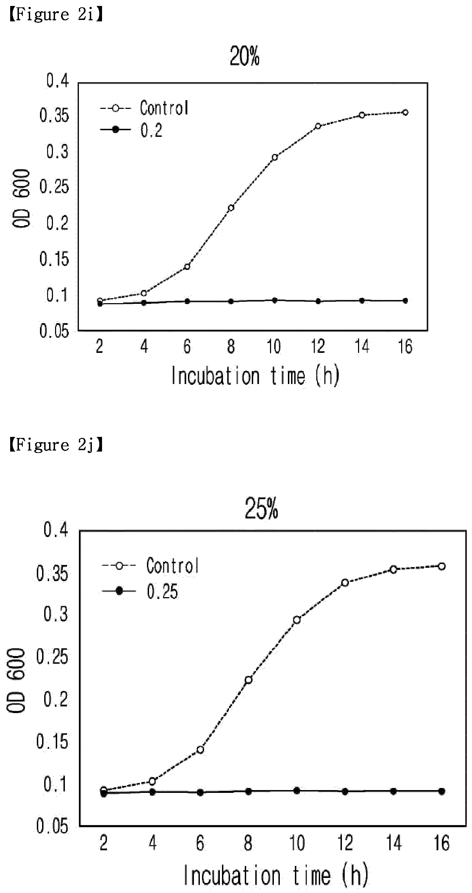

FIGS. 2A to 2J illustrate the results of analyzing the effect of 1,3-propanediol on the growth of the harmful skin bacteria Staphylococcus aureus ATCC 6538. FIGS. 2A to 2J illustrate growth trends through changes in absorbance (OD) values over incubation time of harmful skin bacteria compared to the control group for each treatment concentration of 1,3-propanediol: FIG. 2A: 1,3-propanediol treatment concentration 0.05%; FIG. 2B: 1,3-propanediol treatment concentration 0.1%; FIG. 2C: 1,3-propanediol treatment concentration 0.2%; FIG. 2D: 1,3-propanediol treatment concentration 0.5%; FIG. 2E: 1,3-propanediol treatment concentration 1.0%; FIG. 2F: 1,3-propanediol treatment concentration 3.0%; FIG. 2G: 1,3-propanediol treatment concentration 5.0%; FIG. 2H: 1,3-propanediol treatment concentration 10.0%; FIG. 2I: 1,3-propanediol treatment concentration 20.0%; FIG. 2J: 1,3-propanediol treatment concentration 25.0%.

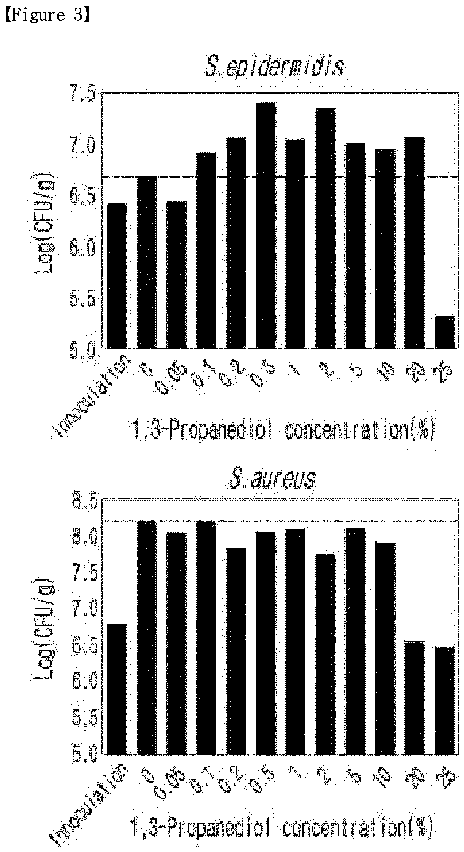

FIG. 3 illustrates the results of analyzing the effect of 1,3-propanediol on growth during co-culture of beneficial and harmful bacteria. FIG. 3A is a graph illustrating the CFU/g values of the beneficial skin bacteria Staphylococcus epidermidis ATCC 12228 converted into log values according to the 1,3-propanediol treatment concentration, and FIG. 3B is a graph illustrating the CFU/g values of the harmful skin bacteria Staphylococcus aureus ATCC 6538 converted into log values according to the 1,3-propanediol treatment concentration.

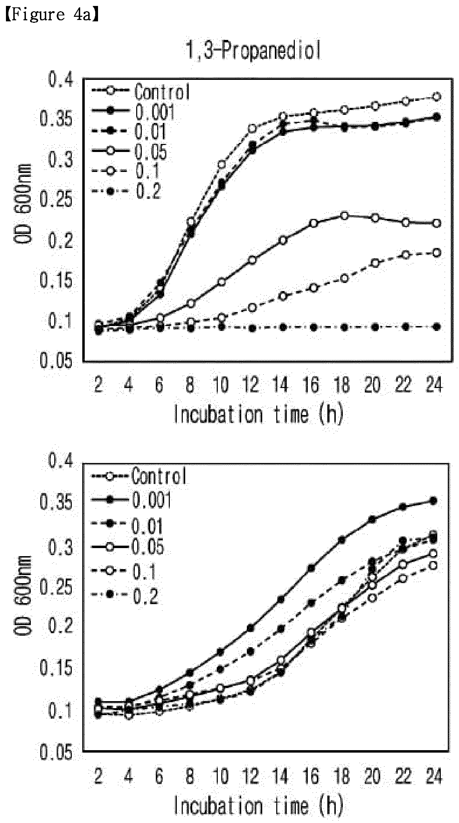

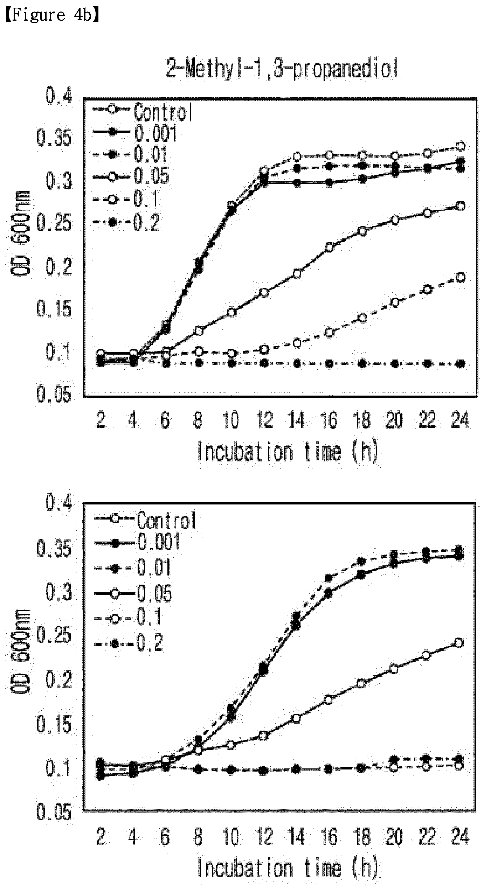

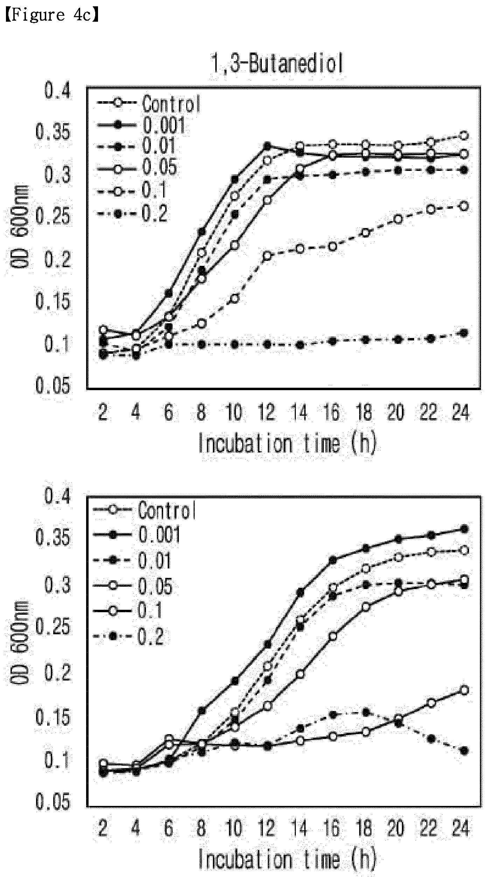

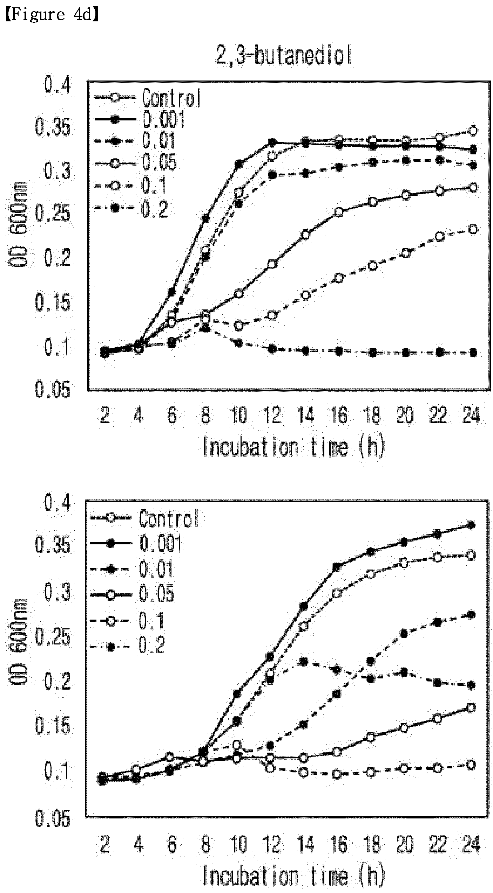

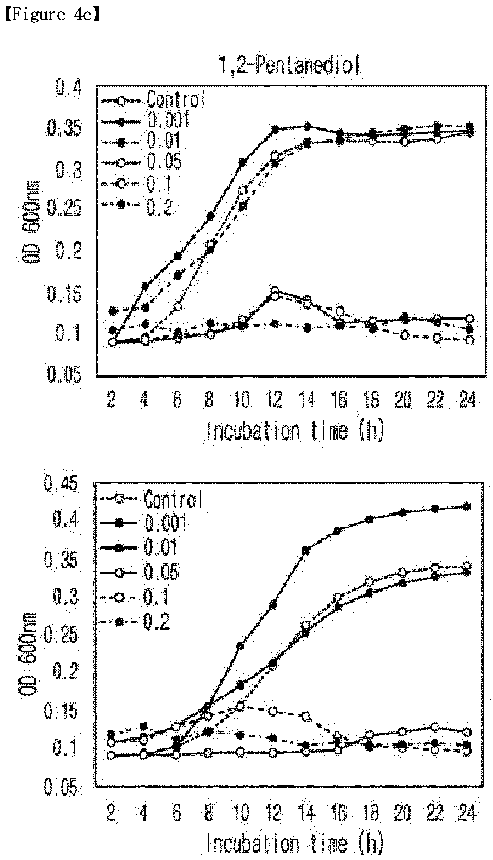

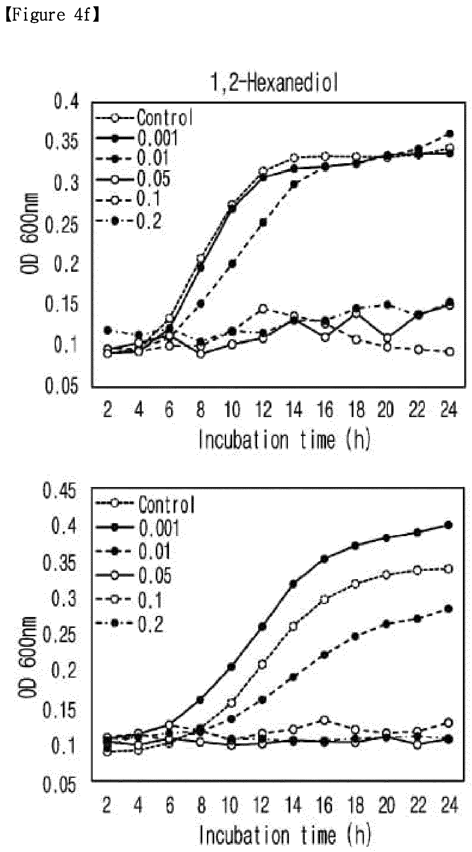

FIG. 4 illustrates the results of a comparative analysis of the effects of diol compounds, specifically 1,3-propanediol, 2-methyl-1,3-propanediol, 1,3-butanediol, 2,3-butanediol, 1,2-pentanediol, and 1,2-hexanediol on the growth of harmful bacteria (upper graph) and beneficial bacteria (lower graph). FIG. 4A: 1,3-propanediol, FIG. 4B: 2-methyl-1,3-propanediol; FIG. 4C: 1,3-butanediol; FIG. 4D: 2,3-butanediol; FIG. 4E: 1,2-pentanediol; FIG. 4F: 1,2-Hexanediol.

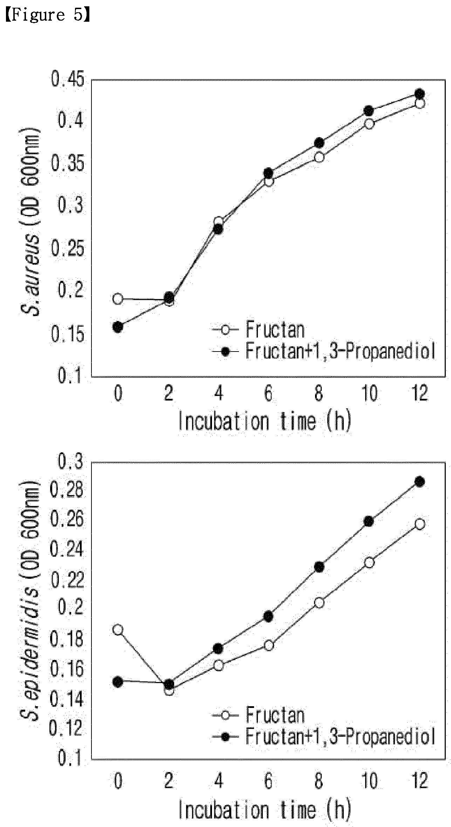

FIG. 5 is a graph illustrating the effect of 1,3-propanediol and fructan in combination on the growth of beneficial and harmful skin bacteria compared to fructan alone. Left: Changes in OD values over incubation time of Staphylococcus aureus after treatment with each sample; Right: Changes in OD values over incubation time of Staphylococcus epidermidis after treatment with each sample.

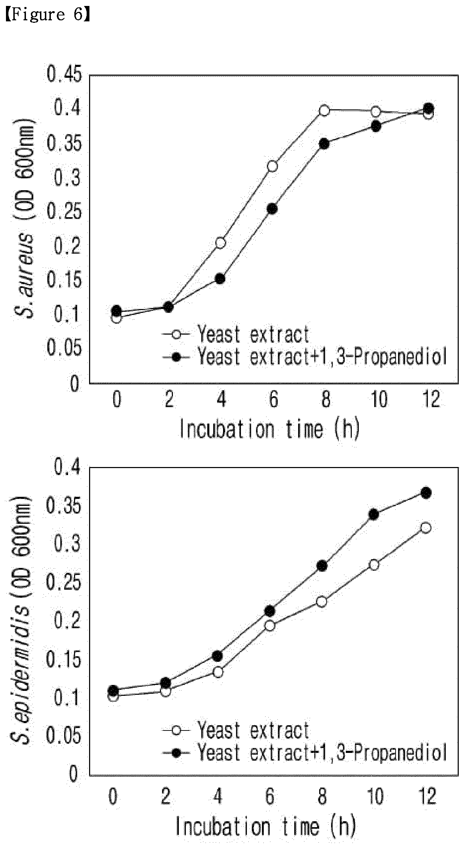

FIG. 6 is a graph illustrating the effect of 1,3-propanediol and yeast extract in combination on the growth of beneficial and harmful skin bacteria compared to yeast extract alone. Left: Changes in OD values over incubation time of Staphylococcus aureus after treatment with each sample; Right: Changes in OD values over incubation time of Staphylococcus epidermidis after treatment with each sample.

### BEST MODE FOR CARRYING OUT THE INVENTION

Hereinafter, embodiments of the present disclosure will be described in detail with reference to the accompanying drawings so that those skilled in the art to which the present disclosure pertains may easily carry out the invention. However, the present disclosure may be embodied in many different forms and is not limited to the embodiments described herein. Throughout the specification, the same reference numerals are used for similar parts.

In addition, in the present disclosure, when similar terms such as "about," "approximately," or "at least" are used in relation to numerical values, it is intended that theoretical, experimental, statistical, or empirical errors of 10%, ±7%, ±5%, ±3%, ±2%, or ±1% are allowed based on the corresponding numerical values.

In one embodiment, the present disclosure provides a composition for skin external use for maintaining the balance of the skin microbiome, containing 1,3-propanediol as an active ingredient.

In the present disclosure, the term "propanediol" refers to a C3 compound in which two hydroxyl groups (-OH) are linked to a saturated hydrocarbon in which carbons form a chain by a single bond, and has two isomers, 1,2- and 1,3-, depending on the position of the hydroxyl group.

The 1,3-propanediol used in the present disclosure is used as a moisturizing ingredient in cosmetics and pharmaceuticals, is also restrictedly used as a food additive, and is amphipathic, including a hydrophilic hydroxyl group and a hydrophobic carbon chain, and has excellent antibacterial properties because it may easily penetrate the cell membrane of microorganisms.

The term "microbiome" as used in the present disclosure refers to a community of various microorganisms such as bacteria and viruses residing in the human body. In addition, in the present disclosure, "microbiome" may be "skin microbiome," which typically refers to microorganisms residing on human skin.

In the present disclosure, "maintaining the balance of the skin microbiome" refers to appropriately maintaining the balance of the microbial community of the skin by relatively increasing (or enhancing) the proliferation and/or activity of beneficial bacteria of the skin while relatively decreasing (or suppressing) the proliferation and/or activity of harmful bacteria of the skin, in order to maintain a healthy skin condition in an individual.

In the present disclosure, "increasing beneficial skin bacteria" refers to relatively promoting the engraftment and/or proliferation of beneficial bacteria that reside on the skin or may be introduced from the outside, and/or relatively activating the growth of beneficial bacteria.

In the present disclosure, "reducing harmful skin bacteria" refers to maintaining the microbial balance of the skin by relatively suppressing the engraftment and/or proliferation of harmful bacteria that reside on the skin or may be introduced from the outside, and/or relatively inhibiting the growth of harmful bacteria.

In the present disclosure, the term "relatively" refers that any environment or condition involved in maintaining the overall balance of the skin microbiome (e.g., temperature, humidity, light, ultraviolet rays, etc.), skin condition (e.g., healthy condition without skin problems, a condition where skin problems have occurred or are at risk of occurring, a condition where the balance of beneficial bacteria and/or harmful bacteria is stable or broken (e.g., due to excessive proliferation and/or death of beneficial bacteria and/or harmful bacteria), etc.), interactions between skin resident bacteria and/or introduced bacteria, substances applied to the skin, and the results caused by the above act favorably on beneficial skin bacteria and unfavorably on harmful skin bacteria.

In the present disclosure, the beneficial bacteria may be one or more selected from the group consisting of Staphylococcus epidermidis, streptococcus pneumoniae, lactobacillus acidophilus, lactobacillus rhamnosus, and bifidobacterium bifidum, specifically, may be Staphylococcus epidermidis, and more specifically, may be Staphylococcus epidermidis ATCC 12228.

For example, Staphylococcus epidermidis is a gram-positive bacterium that generally exists on the skin, mucous membranes, etc. of humans and livestock, and can continue the moisturizing effect of the skin by producing glycerin, maintain the skin acidic by synthesizing organic acids, and suppress the growth of harmful bacteria such as Staphylococcus aureus by producing antibiotics.

In the present disclosure, the harmful bacteria may be one or more selected from the group consisting of Staphylococcus aureus and cutibacterium acnes, specifically, may be Staphylococcus aureus, and more specifically, may be Staphylococcus aureus ATCC 6538.

For example, Staphylococcus aureus is a gram-positive facultative anaerobic bacterium that exists on the skin and nasal cavity surface of healthy humans and livestock, and is known as a causative agent of suppurative diseases such as skin pyoderma, otitis media, and cystitis, as well as food poisoning.

In the present disclosure, the cosmetic composition may contain 1,3-propanediol from 0.1 to 20 wt%, specifically from 1 to 20 wt%, and more specifically from 5 to 20 wt%.

If the content of 1,3-propanediol is less than 0.1 wt%, the growth of beneficial bacteria cannot be promoted, the effect of maintaining the balance of the skin microbiome cannot be exhibited, and ultimately, the effect of improving skin condition cannot be exhibited. If it exceeds 20 wt%, there is a problem that the balance of the skin microbiome is broken by suppressing the activity of both harmful bacteria and beneficial bacteria.

In the present disclosure, the skin external composition may contain 0.5 to 10 wt% of 1,3-propanediol in order to relatively increase the growth of beneficial skin bacteria while relatively inhibiting the growth of harmful skin bacteria without rapidly changing the engraftment, proliferation, death, etc. of beneficial and harmful skin bacteria.

In another embodiment, the present disclosure provides a composition for skin external use for maintaining the balance of the skin microbiome, containing 1,3-propanediol and optionally a boosting ingredient as active ingredients.

In the present disclosure, the boosting ingredient may be at least one selected from the group consisting of fructan and yeast extract.

Fructan is a polymer composed of fructose, and fructans with short chain lengths are known as fructooligosaccharides. Fructan may be found in angiosperms, including monocots and dicots, such as agave, artichoke, asparagus, chives, garlic, onion (including scallions), yacon, jicama, barley, and wheat. It is known to help moisturize the skin and increase skin elasticity, and is listed under CAS No. 9046-37-1 or 639001-44-8.

In the present disclosure, fructan may be extracted and separated using natural materials such as plants known to contain fructan, or commercially available ones may be used.

In the present disclosure, the yeast extract may mean an extract obtained by extracting yeast with a solvent, a fraction obtainable by hydrolyzing yeast cells, a yeast culture medium in which yeast and a culture are present together, an extract extracted from the foregoing fraction or culture medium, a filtrate obtained by filtering yeast from the foregoing fraction or culture medium, a diluted solution or a dried product of the foregoing fraction or extract, and the like. For example, the yeast extract may be a fraction that can be extracted by extracting yeast with a pharmaceutically acceptable organic solvent or separated by a solvent after hydrolyzing yeast cells using an enzyme.

In the present disclosure, the yeast extract may be a beer-derived yeast extract. As the beer-derived yeast extract, those listed under CAS No. 84604-16-0 may be used.

In the present disclosure, the effective concentration of the boosting ingredient may range from 0.06 to 0.25 wt% based on the total weight of the cosmetic composition, preferably from 0.09 to 0.12 wt%.

If the content of the boosting ingredient is less than 0.06 wt%, the growth of beneficial bacteria cannot be promoted, the effect of maintaining the balance of the skin microbiome cannot be exhibited, and ultimately, the effect of improving skin condition cannot be exhibited. If it exceeds 0.25 wt%, there is a problem that the balance of the skin microbiome is broken by suppressing the activity of both harmful bacteria and beneficial bacteria.

In particular, when used in combination with 1,3-propanediol, it may be advantageous to use the boosting ingredient in an amount of 0.09 to 0.12 wt% because it may relatively increase the growth of beneficial skin bacteria while relatively reducing the growth of beneficial skin bacteria without rapidly changing the engraftment, proliferation, death, etc. of beneficial and harmful skin bacteria.

In the skin external composition according to the present disclosure, the combination ratio of 1,3-propanediol and the boosting ingredient may range from 6 to 25:1 by weight, preferably from 12.5 to 16.7:1 by weight.

In the present disclosure, the skin external composition may be formulated as a cosmetic composition or a pharmaceutical composition in a form applicable to the skin.

In the present disclosure, the cosmetic composition may be prepared in a liquid or solid form using bases, adjuvants, and additives commonly used in the cosmetic field. The liquid or solid form of cosmetics may include, for example, forms such as skin toner, cream, lotion, bath agent, and the like, but is not limited thereto. Bases, adjuvants, and additives commonly used in the cosmetic field are not particularly limited, and include, for example, water, alcohol, propylene glycol, stearic acid, glycerol, cetyl alcohol, liquid paraffin, and the like.

The cosmetic composition of the present disclosure may include components commonly used in cosmetic compositions, and may include, for example, conventional auxiliary agents such as antioxidants, stabilizers, solubilizers, vitamins, pigments and fragrances, and carriers.

In the present disclosure, the cosmetic composition may be prepared in any formulation commonly prepared in the art, and may be formulated, for example, as a solution, suspension, emulsion, paste, gel, cream, lotion, powder, soap, surfactant-containing cleanser, oil, microcapsule, microgranule, ionic vesicle dispersion, nonionic vesicle dispersion, powder foundation, emulsion foundation, wax foundation, stick, mask pack, and spray, but is not limited thereto. More specifically, it may be prepared in formulations of emollient toner, nutrient toner, nutrient cream, massage cream, essence, eye cream, cleansing cream, cleansing foam, cleansing water, pack, spray, or powder.

In the present disclosure, when the formulation of the cosmetic composition is paste, cream, or gel, the followings may be used as a carrier component: animal oil, vegetable oil, wax, paraffin, starch, tragacanth, cellulose derivative, polyethylene glycol, silicone, bentonite, silica, talc, or zinc oxide.

In the present disclosure, when the formulation of the cosmetic composition is powder or spray, the followings may be used as a carrier component: lactose, talc, silica, aluminum hydroxide, calcium silicate, or polyamide powder. And in the case of spray, in particular, a propellant such as chlorofluorohydrocarbon, propane/butane, or dimethyl ether may be additionally included.

In the present disclosure, when the formulation of the cosmetic composition is a solution or emulsion, the followings may be used as a carrier component: a solvent, a solubilizer, or an emulsifier. And for example, water, ethanol, isopropanol, ethyl carbonate, ethyl acetate, benzyl alcohol, benzyl benzoate, glycerol aliphatic ester, polyethylene glycol, or fatty acid ester of sorbitan may be used.

In the present disclosure, when the formulation of the cosmetic composition is a suspension, the followings may be used as a carrier component: a liquid diluent such as water or ethanol, a suspending agent such as ethoxylated isostearyl alcohol, polyoxyethylene sorbitol ester and polyoxyethylene sorbitan ester, microcrystalline cellulose, aluminum metahydroxide, bentonite, agar or tragacanth.

In the present disclosure, when the formulation of the cosmetic composition is a surfactant-containing cleanser, the followings may be used as a carrier component: an aliphatic alcohol sulfate, an aliphatic alcohol ether sulfate, a sulfosuccinic acid monoester, an imidazolinium derivative, a methyl taurate, a sarcosinate, a fatty acid amide ether sulfate, an alkylamidobetaine, a fatty alcohol, a fatty acid glyceride, a fatty acid diethanolamide, vegetable oil, lanolin derivative or ethoxylated glycerol fatty acid ester.

The cosmetic composition of the present disclosure may be used by applying it alone or in multiple applications, or by applying it in multiple applications with other cosmetic compositions other than the present disclosure. In addition, the cosmetic composition of the present disclosure may be used according to a common usage method, and the number of uses may be varied depending on the user's skin condition or preference.

In the present disclosure, when the cosmetic composition is in the form of soap, a surfactant-containing cleansing agent, or a surfactant-free cleansing agent, it may be applied to the skin and then wiped off, removed, or washed off with water. For example, soap is liquid soap, powdered soap, solid soap, and oil soap, surfactant-containing cleansing formulations are cleansing foam, cleansing water, cleansing towel, and cleansing pack, and surfactant-free cleansing formulations are cleansing cream, cleansing lotion, cleansing water, and cleansing gel, and are not limited thereto.

In the present disclosure, the pharmaceutically acceptable composition in a form applicable to the skin may further include a pharmaceutically acceptable carrier, excipient, or diluent, if necessary, in addition to the foregoing active ingredient and boosting ingredient. The pharmaceutically acceptable carrier, excipient, or diluent is not limited as long as it can achieve the effects of the composition according to the present disclosure, and may include, for example, fillers, extenders, binders, humectants, disintegrants, surfactants, lubricants, sweeteners, flavoring agents, and preservatives.

Representative examples of the pharmaceutically acceptable carrier, excipient, or diluent may include lactose, dextrose, sucrose, sorbitol, mannitol, xylitol, maltitol, starch, gelatin, glycerin, acacia gum, alginate, calcium phosphate, calcium carbonate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, propylene glycol, polyethylene glycol, vegetable oil, injectable ester, witepsol, macrogol, tween 61, cocoa butter, lauryl alcohol, and the like, and are not particularly limited thereto.

The pharmaceutical composition of the present disclosure may be prepared as a common quasi-drug. Examples of such quasi-drugs include, but are not limited to, liquid, powder, ointment, lotion, wet wipe, spray, bandage, or patch, and the preparation method, dosage, method of use, constituent components, etc. of quasi-drugs may be appropriately selected by those skilled in the art from conventional techniques known in the art.

The pharmaceutical composition of the present disclosure may additionally include one or more active ingredients that may exhibit the same or similar functions as the foregoing active ingredient. The additional active ingredient may be selected in consideration of synergistic effects, skin safety, ease of formulation, and the like due to combined use. The additional active ingredient may be included at 0.0001 to 10 wt% (w/w) based on the total weight of the quasi-drug composition, and the content range may be appropriately adjusted by those skilled in the art in consideration of skin safety, ease of formulation, and the like.

In addition, the pharmaceutical composition according to the present disclosure may further include other components or substances for imparting additional effects such as skin whitening, trouble improvement, anti-inflammatory, moisturizing, skin wrinkle improvement, and enhancement of antioxidant activity in addition to the main effects exhibited by the active ingredient.

### MODE FOR CARRYING OUT THE INVENTION

Hereinafter, the configuration and effects of the present disclosure will be described in more detail through examples. These examples are only for illustrating the present disclosure, and the scope of the present disclosure is not limited by these examples.

### <Preparation of strains and samples>

1) Staphylococcus epidermidis ATCC 12228 and Staphylococcus aureus ATCC 6538 strains were obtained from ThermoFisher Scientific and cultured in tryptic soy broth (culture conditions: 32°C for 18 hours). After culturing, they were diluted with 0.85% NaCl to adjust the absorbance (600 nm) to 0.5-0.6.
2) 1,3-propanediol was obtained from Sigma-Aldrich.
3) 2-methyl-1,3-propanediol, 1,3-butanediol, 2,3-butanediol, 1,2-pentanediol, and 1,2-hexanediol were all obtained from Sigma-Aldrich.
4) Fructan was obtained from Sigma-Aldrich.
5) Yeast extract was obtained from Sigma-Aldrich.

### <Experimental Example 1> Analysis of the effect of 1,3-propanediol on beneficial skin bacteria (growth rate)

The effect of 1,3-propanediol on the growth of Staphylococcus epidermidis ATCC 12228 was analyzed.

For this purpose, 1,3-propanediol was diluted with 10% tryptic soy broth to prepare concentrations of 0.05, 0.1, 0.2, 0.5, 1.0, 3.0, 5.0, 10, 20, and 25 wt%.

After adding the prepared 1,3-propanediol at each concentration to 10% tryptic soy broth, *S. epidermidis* was inoculated to achieve a concentration of 1% (v/v).

Afterwards, the cells were cultured at a temperature of 32°C for 24 hours, and the absorbance (600 nm) was measured at 2-hour intervals to analyze the growth of beneficial skin bacteria.

At this time, 10% tryptic soy broth to which 1,3-propanediol was not added was set as a control group.

Table 1 below shows the results of analyzing the degree of *S. epidermidis* growth according to the concentration of 1,3-propanediol.

**[Table 1]**

| Concentration of 1,3-propanediol | Effect on the growth of beneficial bacteria (*S. epidermidis*) |
|---|---|
| 0.05 wt% | No effect |
| 0.1 wt% | Promoted |
| 0.2 wt% | Promoted |
| 0.5 wt% | Promoted |
| 1.0 wt% | Promoted |
| 3.0 wt% | Promoted |
| 5.0 wt% | Promoted |
| 10 wt% | Promoted |
| 20 wt% | No effect |
| 25 wt% | Inhibited |

The criteria for determining the effect on the growth of beneficial bacteria were as follows: when the absorbance (600 nm) was measured while culturing for 2 to 16 hours after inoculation of *S. epidermidis,* if the absorbance was higher than that of 10% tryptic soy broth (control group) to which 1,3-propanediol was not added, it was determined that the growth of S. epidermidis was promoted. If there was no difference, it was determined that there was no effect, and if the absorbance was lower, it was determined that there was inhibition. Referring to FIG. 1 and Table 1, 1,3-propanediol with a concentration of 0.05 wt% or less did not promote the growth of *S. epidermidis,* and it can be seen that when the concentration was 0.1 wt% or more, it promoted the growth of *S. epidermidis.* In addition, it can be seen that when the concentration was 20 wt%, it did not promote the growth of *S*. *epidermidis,* and when it exceeded 25 wt%, it inhibited the growth of *S. epidermidis.* As a result, it can be seen that when the concentration of 1,3-propanediol is in a specific range exceeding 0.05 wt% and less than 20 wt%, it promotes the growth of *S. epidermidis.*

### <Experimental Example 2> Analysis of the effect of 1,3-propanediol on harmful skin bacteria (growth rate)

The effect of 1,3-propanediol on the growth of Staphylococcus aureus ATCC 6538 was analyzed.

For this purpose, 1,3-propanediol was diluted with 10% tryptic soy broth to prepare concentrations of 0.05, 0.1, 0.2, 0.5, 1.0, 3.0, 5.0, 10, 20, and 25 wt%.

After adding 1,3-propanediol prepared at each concentration to 10% tryptic soy broth, *S. aureus* was inoculated to achieve a concentration of 1% (v/v).

Afterwards, the cells were cultured at a temperature of 32°C for 24 hours, and the absorbance (600 nm) was measured at 2-hour intervals to analyze the growth of beneficial skin bacteria.

At this time, 10% tryptic soy broth to which 1,3-propanediol was not added was set as a control group.

**[Table 2]**

| Concentration of 1,3-propanediol | Effect on the growth of harmful bacteria (*S. aureus*) |
|---|---|
| 0.05 wt% | No effect |
| 0.1 wt% | No effect |
| 0.2 wt% | No effect |
| 0.5 wt% | No effect |
| 1.0 wt% | No effect |
| 3.0 wt% | Inhibited |
| 5.0 wt% | Inhibited |
| 10 wt% | Inhibited |
| 20 wt% | Inhibited |
| 25 wt% | Inhibited |

The criteria for determining the effect on the growth of harmful bacteria were as follows: when the absorbance (600 nm) was measured while culturing for 2 to 16 hours after inoculation of *S. aureus,* if the absorbance was lower than that of 10% tryptic soy broth (control group) to which 1,3-propanediol was not added, it was determined that the growth of *S. aureus* was inhibited. If there was no difference in absorbance, it was determined that there was no effect. Referring to FIG. 2 and Table 2, when the concentration of 1,3-propanediol was 1.0 wt% or less, it did not inhibit the growth of *S*. *aureus,* and when the concentration of 1,3-propanediol was 3.0 wt% or more, it can be seen that it inhibited the growth of *S. aureus.*

As a result, it can be seen that when the concentration of 1,3-propanediol is 3.0 wt% or more, it inhibits the growth of *S. aureus.*

### <Experimental Example 3> Analysis of the growth of beneficial and harmful bacteria during co-culture

The effect of 1,3-propanediol on beneficial and harmful skin bacteria during co-culture was analyzed.

For this purpose, 1,3-propanediol was diluted with 10% tryptic soy broth to prepare concentrations of 0.05, 0.1, 0.2, 0.5, 1.0, 3.0, 5.0, 10, 20, and 25 wt%.

After adding 1,3-propanediol prepared at each concentration to 10% tryptic soy broth, *S. epidermidis* and *S. aureus* were each inoculated to achieve a concentration of 1% (v/v).

Afterwards, a 0.4 µm insert was used in a 12-well plate to create a state where beneficial bacteria and harmful bacteria were cultured independently while sharing metabolites.

After culturing at 32°C for 6 hours, the number of viable bacteria was measured to analyze the effect of 1,3-propanediol on beneficial and harmful skin bacteria.

At this time, 10% tryptic soy broth to which 1,3-propanediol was not added was used as a control group.

Referring to FIG. 3, it was confirmed that the concentration of 1,3-propanediol that promotes the growth of beneficial bacteria is from 0.1 wt% to 20 wt%, and the concentration of 1,3-propanediol that inhibits the growth of harmful bacteria is from 0.05 wt% to 25 wt%.

Therefore, when the concentration of 1,3-propanediol is 0.1 to 20 wt%, it can maintain the balance of the skin microbiome by promoting the growth of beneficial bacteria and inhibiting the growth of harmful bacteria. In particular, the concentration of 1,3-propanediol that is preferable for relatively increasing the growth rate of beneficial bacteria compared to the growth rate of harmful bacteria without rapid changes in beneficial bacteria and harmful bacteria is 0.2 to 10 wt%.

### <Experimental Example 4> Comparison of the microbiome balancing effect of various diol compounds

The microbiome balancing effect of 1,3-propanediol was compared with other diol compounds and is shown in Tables 3 and 4 below.

**[Table 3]**

| Classification | Diol compound concentration | | | | |
|---|---|---|---|---|---|
| | Effect on the growth of harmful bacteria *(S. aureus)* | | | | |
| | 0.1 wt% | 1 wt% | 5 wt% | 10 wt% | 20 wt% |
| 1,3-propanediol | ++ | ++ | +++ | +++ | +++ |
| 2-methyl-1,3-propanediol | + | + | ++ | +++ | +++ |
| 1,3-butanediol | + | + | ++ | ++ | +++ |
| 2,3-butanediol | + | + | ++ | +++ | +++ |
| 1,2- | + | + | +++ | +++ | +++ |
| pentanediol | | | | | |
| 1,2-hexanediol | + | ++ | +++ | +++ | +++ |

Effect on the growth of *S. aureus* - No effect: +; Growth inhibition: ++; 50% or more inhibition: +++

**[Table 4]**

| Classification | Diol compound concentration | | | | |
|---|---|---|---|---|---|
| | Effect on the growth of beneficial bacteria (*S. epidermidis*) | | | | |
| | 0.1 wt% | 1 wt% | 5 wt% | 10 wt% | 20 wt% |
| 1,3-propanediol | ++ | ++ | + | + | + |
| 2-methyl-1,3-propanediol | + | + | - | - - | - - |
| 1,3-butanediol | ++ | - | - | - - | - - |
| 2,3-butanediol | ++ | - | - - | - - | - - |
| 1,2-pentanediol | ++ | - | - - | - - | - - |
| 1,2-hexanediol | ++ | - | - - | - - | - - |

Effect on the growth of *S. epidermidis* - Growth promotion: ++; No effect: +; Growth inhibition: -; 50% or more growth inhibition: - -

Referring to FIG. 4, Tables 3 and 4, as a result of comparing the growth inhibitory effect of harmful bacteria, it can be seen that all diol compounds including 1,3-propanediol of the present disclosure inhibit the growth of harmful bacteria at similar concentrations.

However, as a result of comparing the growth effect of beneficial bacteria, it can be seen that other diol compounds other than 1,2-propanediol inhibit the growth of beneficial bacteria when the concentration exceeds 0.1 wt%.

In other words, in the case of general diol chemicals, when the concentration increases, it inhibits the growth of both beneficial and harmful bacteria, whereas in the case of 1,3-propanediol, it can be seen that the growth of beneficial bacteria is promoted and the growth of harmful bacteria is inhibited.

As a result, it can be seen that 1,3-propanediol can promote the growth of beneficial bacteria and inhibit the activity of harmful bacteria at a specific concentration, so it can be applied as a composition for maintaining the balance of the skin microbiome.

### <Experimental Example 5> Evaluation of the effect on the skin microbiome by using a combination of 1,3-propanediol and fructan

In order to evaluate the effect of 1,3-propanediol and fructan on the skin microbiome, the effect on the growth rate of Staphylococcus epidermidis ATCC 12228, a representative beneficial skin bacterium, and Staphylococcus aureus ATCC 6538, a harmful skin bacterium, was confirmed. Staphylococcus epidermidis ATCC 12228 and Staphylococcus aureus ATCC 6538 were cultured in tryptic soy broth at 32°C for 18 hours or more, then diluted with 0.85% NaCl and adjusted to have an absorbance (600 nm) of 0.5 to 0.6.

*S. epidermidis* and *S. aureus* prepared above were inoculated to achieve a concentration of 1% (v/v) in 10% tryptic soy broth to which 1,3-propanediol was added at a fixed concentration of 1.5 wt% and fructan was added at each concentration in the table below. The degree of growth of beneficial and harmful skin bacteria was compared by measuring the absorbance (600 nm) at 2-hour intervals while culturing at 32°C for 12 hours.

The results are shown in Tables 5 and 6.

**[Table 5]**

| Classification | | Fructan concentration (wt%) | | | | | | | |
|---|---|---|---|---|---|---|---|---|---|
| | | Effect on the growth of harmful bacteria (*S. aureus*) | | | | | | | |
| | | 0.00 | 0.04 | 0.06 | 0.09 | 0.10 | 0.12 | 0.25 | 0.30 |
| 1,3-propanediol | 0 wt% | * | * | * | * | * | * | * | * |
| | 1.5 wt% | ++ | ++ | ++ | ++ | ++ | ++ | ++ | ++ |

(Criteria for evaluating the effect on the growth of *S. aureus*) No noticeable effect (change in absorbance within 1%): *; Mild growth inhibition (change in absorbance (decrease) 3%): +; Significant growth inhibition (change in absorbance (decrease) 5%): ++; Severe growth inhibition (change in absorbance (decrease) 10% or more): +++

**[Table 6]**

| Classification | | Fructan concentration (wt%) | | | | | | | |
|---|---|---|---|---|---|---|---|---|---|
| | | Effect on the growth of beneficial bacteria (*S. epidermidis*) | | | | | | | |
| | | 0.00 | 0.04 | 0.06 | 0.09 | 0.10 | 0.12 | 0.25 | 0.30 |
| 1,3-propanediol | 0 wt% | * | * | + | + | + | + | ++ | ++ |
| | 1.5 wt% | + | + | ++ | +++ | +++ | +++ | +++ | +++ |

(Criteria for evaluating the effect on the growth of *S. epidermidis*) No noticeable effect (change in absorbance within 1%): *; Mild growth promotion (change in absorbance (increase) 3%): +; Significant growth promotion growth inhibition (change in absorbance (increase) 5%): ++; Severe growth promotion (change in absorbance (increase) 10% or more): +++; Mild growth inhibition (change in absorbance (decrease) 3% or more): -; Significant growth inhibition (change in absorbance (decrease) 5% or more): - -

From Tables 5 and 6 above, if the content of fructan is less than 0.06 wt%, the growth of beneficial bacteria cannot be promoted, and if it exceeds 0.25 wt%, there is a problem that the balance of the skin microbiome is broken by suppressing the activity of both harmful bacteria and beneficial bacteria.

In addition, when treated with 0.1 wt% of fructan and 1.5 wt% of 1,3-propanediol compared to 0.3 wt% of fructan, it was confirmed that there was no significant effect on inhibiting the growth of harmful bacteria as the incubation time increased, but the efficacy of promoting the growth of beneficial bacteria was relatively increased (see FIG. 5).

This is considered to be a result of fructan boosting the beneficial bacteria growth promoting efficacy of 3-propanediol.

### <Experimental Example 6> Evaluation of the effect on the skin microbiome by using a combination of 1,3-propanediol and yeast extract

The degree of growth of beneficial and harmful skin bacteria was compared in the same manner as in <Experimental Example 5> to evaluate the effect of 1,3-propanediol and yeast extract on the skin microbiome.

The results are shown in Tables 7 and 8.

**[Table 7]**

| Classification | | Yeast extract concentration (wt%) | | | | | | | |
|---|---|---|---|---|---|---|---|---|---|
| | | Effect on the growth of harmful bacteria (*S. aureus*) | | | | | | | |
| | | 0.00 | 0.04 | 0.06 | 0.09 | 0.10 | 0.12 | 0.25 | 0.30 |
| 1,3-propanediol | 0 wt% | * | * | * | * | * | * | * | * |
| | 1.5 wt% | ++ | ++ | ++ | ++ | ++ | ++ | ++ | ++ |

(Criteria for evaluating the effect on the growth of *S. aureus*) No noticeable effect (change in absorbance within 1%): *; Mild growth inhibition (change in absorbance (decrease) 3%): +; Significant growth inhibition (change in absorbance (decrease) 5%): ++; Severe growth inhibition (change in absorbance (decrease) 10% or more): +++

**[Table 8]**

| Classification | | Yeast extract concentration (wt%) | | | | | | | |
|---|---|---|---|---|---|---|---|---|---|
| | | Effect on the growth of beneficial bacteria (*S. epidermidis*) | | | | | | | |
| | | 0.00 | 0.04 | 0.06 | 0.09 | 0.10 | 0.12 | 0.25 | 0.30 |
| 1,3-propanediol | 0 wt% | * | * | + | + | + | + | ++ | ++ |
| | 1.5 wt% | + | + | ++ | +++ | +++ | +++ | +++ | +++ |

(Criteria for evaluating the effect on the growth of *S. aureus*) No noticeable effect (change in absorbance within 1%): *; Mild growth promotion (change in absorbance (increase) 3%): +; Significant growth promotion growth inhibition (change in absorbance (increase) 5%): ++; Severe growth promotion (change in absorbance (increase) 10% or more): +++; Mild growth inhibition (change in absorbance (decrease) 3% or more): -; Significant growth inhibition (change in absorbance (decrease) 5% or more): - -

From Tables 7 and 8 above, if the content of yeast extract is less than 0.06 wt%, the growth of beneficial bacteria cannot be promoted, and if it exceeds 0.25 wt%, there is a problem that the balance of the skin microbiome is broken by suppressing the activity of both harmful bacteria and beneficial bacteria.

In addition, when treated with 0.1 wt% of yeast extract and 1.5 wt% of 1,3-propanediol compared to 0.3 wt% of yeast extract, it was shown that the growth promotion efficacy of beneficial bacteria also increased along with the suppression of harmful bacteria growth as the incubation time increased, but it was confirmed that the growth promotion effect of beneficial bacteria was relatively greater (see FIG. 6).

This is considered to be a result of yeast extract boosting the beneficial bacteria growth promoting efficacy of 3-propanediol.

### INDUSTRIAL APPLICABILITY

In the present disclosure, it was confirmed that only 1,3-propanediol exhibits the effect of maintaining the balance of the skin microbiome compared to other diol compounds commonly used in the industry, and surprisingly, it was clarified that it can exhibit a significant action effect when the concentration is 0.1 to 20 wt%. In addition, there is no particular problem in formulating it into a pharmaceutical composition such as a cosmetic composition or a quasi-drug for external use on the skin, so it has high industrial value.

The skin external composition according to the present disclosure is formulated as a cosmetic composition or a pharmaceutical composition such as a quasi-drug for external use on the skin, and can help maintain the skin health of an individual, so it is industrially valuable.

The skin external composition according to the present disclosure, by including 1,3-propanediol as an active ingredient, promotes the growth of beneficial skin bacteria and suppresses the growth of harmful skin bacteria to maintain the balance of the skin microbiome, thereby improving and strengthening the barrier function against external irritants of the skin. It has high utility value because it can help to make the skin tissue firm, improve skin texture, and maintain overall skin health, and has the advantage of contributing to improving the quality of life by boosting the user's confidence in their skin.

## Claims

1. A composition for skin external use for maintaining the balance of the skin microbiome, comprising 1,3-propanediol as an active ingredient and optionally a boosting ingredient.

2. The composition of claim 1, wherein maintaining the balance of the microbiome refers to increasing beneficial bacteria while decreasing harmful bacteria in the skin.

3. The composition of claim 2, wherein,
the beneficial bacteria are one or more selected from the group consisting of StaphylococcusStaphylococcus epidermidis, Streptococcus pneumoniae, Latobacillus acidophilus, Lacotbacillus rhamnosus, and Bifidobacterium bifidum, and
the harmful bacteria are one or more selected from the group consisting of Staphylococcus aureus and cutibacterium acnes.

4. The composition of claim 1, wherein the effective concentration of 1,3-propanediol in the composition for skin external use ranges from 0.1 to 20 wt%.

5. The composition of claim 1, wherein the boosting ingredient is at least one selected from the group consisting of fructan and yeast extract.

6. The composition of claim 1, wherein the effective concentration of the boosting ingredient in the composition for skin external use ranges from 0.06 to 0.25 wt%, or from 0.09 to 0.12 wt%.

7. The composition of claim 1, wherein the combination ratio of 1,3-propanediol and the boosting ingredient ranges from 6 to 25:1 by weight, or from 12.5 to 16.7:1 by weight.

8. The composition of claim 1, wherein the composition is formulated into a cosmetic composition or a pharmaceutical composition in a form topically applicable to the skin.

9. The composition of claim 8, wherein,
the cosmetic composition comprises a cosmetically acceptable carrier, excipient, diluent, or a mixture thereof, and
the cosmetic composition is formulated into a form selected from the group consisting of solution, suspension, emulsion, paste, gel, cream, lotion, powder, soap, surfactant-containing cleanser, oil, microcapsule, microgranule, ionic vesicle dispersion, non-ionic vesicle dispersion, powder foundation, emulsion foundation, wax foundation, stick, mask pack, and spray.

10. The composition of claim 8, wherein,
the pharmaceutical composition comprises a pharmaceutically acceptable carrier, excipient, diluent, or a mixture thereof, and
the pharmaceutical composition is formulated into a form selected from the group consisting of liquid, powder, ointment, lotion, wet tissue, spray, bandage, and patch.

11. A method for maintaining or restoring the balance of the skin microbiome by topically applying to the skin a skin external composition to the skin comprising 1,3-propanediol or a combination of 1,3-propanediol and a boosting ingredient as an active ingredient.

12. The method according to Claim 11, wherein the boosting ingredient is at least one selected from the group consisting of fructan and yeast extract.

13. The method according to Claim 11, wherein the effective concentration of the boosting ingredient in the composition for skin external use ranges from 0.06 to 0.25 wt%*,* or from 0.09 to 0.12 wt%.

14. The method according to Claim 11, wherein the combination ratio of 1,3-propanediol and the boosting ingredient ranges from 6 to 25:1 by weight, or from 12.5 to 16.7:1 by weight.

15. Use of 1,3-propanediol or a combination of 1,3-propanediol and a boosting ingredient as an active ingredient in a skin external composition for maintaining the balance of the skin microbiome.

16. The method according to Claim 15, wherein the boosting ingredient is at least one selected from the group consisting of fructan and yeast extract.

17. The method according to Claim 15, wherein the effective concentration of the boosting ingredient in the composition for skin external use ranges from 0.06 to 0.25 wt%, or from 0.09 to 0.12 wt%.

18. The method according to Claim 15, wherein the combination ratio of 1,3-propanediol and the boosting ingredient ranges from 6 to 25:1 by weight, or from 12.5 to 16.7:1 by weight.
